# EUROPEAN PATENT APPLICATION

(11) **EP 4 342 846 A1**
(43) Date of publication of application: **27.03.2024**
(21) Application number: 22196566.8
(22) Date of filing: 20.09.2022
(51) Int. Cl.: C01B 32/50, C01B 3/00, C07C 51/00, F17C 5/06, C25B 1/04

(54) **CONTINUOUS PRODUCTION OF PRESSURIZED CARBON DIOXIDE**

(71) Applicant: Evonik Operations GmbH, 45128 Essen (DE)
(72) Inventor: The designation of the inventor has not yet been filed
(74) Representative: Evonik Patent Association

(57) **Abstract**

The present invention relates to a method and a reactor system for the continuous production of pressurized carbon dioxide. The continuous production of carbon dioxide is achieved by the release of carbon dioxide from a carbon dioxide releasing carrier material to thereby obtain compressed products comprising pressurized carbon dioxide and the carrier material. The obtained carrier material can react with further carbon dioxide to obtain the carbon dioxide releasing carrier material used in the releasing step.

## Description

### TECHNICAL FIELD

The present invention relates to a method and a reactor system for the continuous production of pressurized carbon dioxide. The continuous production of carbon dioxide is achieved by the release of carbon dioxide from a carbon dioxide releasing carrier material to thereby obtain compressed products comprising pressurized carbon dioxide and the carrier material. The obtained carrier material can react with further carbon dioxide to obtain the carbon dioxide releasing carrier material used in the releasing step.

### TECHNICAL BACKGROUND

Carbon dioxide is predominantly an unrecovered waste product obtained from industrial processes or from the combustion of carbon-based fuels. Pressurized carbon dioxide is useful for chemical reactions and technical processes as well as in the pharmaceutical, healthcare and food industry. For instance, pressurized carbon dioxide, such as liquid carbon dioxide, is excellent for the preservation of food, fire extinguishers, and in commercial food processes. Furthermore, due to climate change, great efforts are being taken worldwide to limit in the future the release of carbon dioxide into the atmosphere. One possible way to do so is to capture the produced carbon dioxide from the emitting sources (like power plants or heavy industry) and store the gas in geological formations several hundred meters below ground or even in sediments below sea bottom. This so-called carbon capture and storage (CCS) needs compression of carbon dioxide in order to store it in underground deposits.

Particularly, supercritical carbon dioxide can be used as solvent, working fluid, sterilization or cleaning material. However, for the provision of supercritical carbon dioxide or pressurized carbon dioxide, the carbon dioxide needs to be compressed. For instance, the critical pressure that is required for obtaining supercritical carbon dioxide is about 73.8 bar.

Numerous compressors for obtaining pressurized or compressed carbon dioxide are known in the prior art. Particularly, mechanical hydrogen compressors are conventionally used such as piston compressors, turbine engine compressors, membrane compressors, or centrifugal compressors. During compression using mechanical processes or apparatus, the temperature of the carbon dioxide rises due to the thermodynamic nature of compression, i.e. irreversible thermodynamic change of state of the gas. The major part of the energy that is required for the mechanical compression of the carbon dioxide is thus converted to heat. The heat has to be dissipated to the environment and is lost to the process. This leads to a very high energy demand for mechanical compression of carbon dioxide. Mechanical compressors for high pressure compression consist of several stages to limit these losses, by cooling the compressed carbon dioxide between the stages, which leads to a high complexity of these machines. Furthermore, mechanical compressors need various means for cooling. This is particularly relevant for an industrial scaled production of compressed carbon dioxide, where a huge amount of heat must be removed, e.g. by a cooling tower. Consequently, additional energy is needed for the cooling process that is particularly undesired for an industrial scaled production for compressed hydrogen.

Accordingly, it is necessary to provide a new and inventive method as well as a system for a supply of pressurized carbon dioxide that requires less energy for the compression of the carbon dioxide by reducing the energy dissipated to the environment and is environmentally friendly.

The present invention provides a new and inventive method as well as reactor system for the continuous compression of carbon dioxide. The compression of the carbon dioxide is achieved by releasing carbon dioxide gas (or supercritical carbon dioxide) from a carbon dioxide releasing carrier material in a reactor. Then, the carrier material is transferred into another reactor and oxidized in order to obtain the aforementioned carbon dioxide releasing carrier material. Accordingly, a continuous process is disclosed that continuously produce compressed or pressurized carbon dioxide.

### SUMMARY OF THE INVENTION

Particularly, this objective can be achieved by the method according to the invention for the continuous production of pressurized carbon dioxide comprising (a) reacting, such as reducing, particularly hydrogenating, electrochemically reducing and/or hydrothermal reducing, the carbon dioxide in a first reactor, particularly a pressure regulated first reactor, to obtain a carbon dioxide releasing carrier material, (b) transferring the carbon dioxide releasing carrier material to a second reactor, particularly a pressure regulated second reactor, (c) releasing carbon dioxide from the carbon dioxide releasing carrier material in a second reactor to thereby obtain compressed products comprising pressurized carbon dioxide and the carrier material, (d) separating the compressed products comprising pressurized carbon dioxide and the carrier material obtained in step (c) to obtain pressurized carbon dioxide, and (e) optionally supplying the first reactor with the carrier material, such as the separated carrier material, obtained in step (d) for the reaction, such as reducing, particularly hydrogenating, electrochemically reducing and/or hydrothermal reducing, in step (a).

The present invention also concerns a reactor system for the continuous production of pressurized carbon dioxide comprising (a) a first reactor, particularly a pressure regulated first reactor, for the reaction, such as reduction, particularly hydrogenation, electrochemically reduction and/or hydrothermal reduction, of carbon dioxide to obtain a carbon dioxide releasing carrier material, (b) a first connecting element for the transfer of the carbon dioxide releasing carrier material from the first reactor to a second reactor, (c) a second reactor, particularly a pressure regulated second reactor, for the releasing of the carbon dioxide from the carbon dioxide releasing carrier material to thereby obtain compressed products comprising pressurized carbon dioxide and the carrier material, (d) a separator in connection with the second reactor (c) for the separation of the compressed products comprising the pressurized carbon dioxide and the carrier material, (e) optionally a second connecting element connected to the separator for the transfer of the carrier material from the separator (d) to the first reactor (a).

Specific or preferred variants of the method or reactor system of the present invention are set forth in the dependent claims, aspects and figures. The present invention also concerns pressurized carbon dioxide produced according to the method according to any one of the preceding claims/aspects and/or using a reactor system according to any one of the preceding claims/aspects. Moreover, the present invention relates to the use of a carrier material for the continuous production of pressurized carbon dioxide. These and other optional features and advantages of the present invention are described in more detail in the following description, aspects and figures.

### BRIEF DESCRIPTION OF THE DRAWINGS

Figure 1: Flow-Chart for the continuous production of pressurized carbon dioxide.
Figure 2: Reactor system for the continuous production of pressurized carbon dioxide.

### DETAILED DESCRIPTION

The present invention relates to a method and a reactor system for the continuous production of pressurized carbon dioxide.

The terms compressed and pressurized with respect to carbon dioxide or hydrogen are used synonymously. The continuous production means that the method/reactor system is able to constantly produce pressurized carbon dioxide, wherein the carrier material successively reacts to the carbon dioxide releasing carrier material and after the release of the carbon dioxide, the initial carrier material is obtained that can be either resupplied to the first reactor or collected as pressurized carrier material, such as hydrogen. Generally, a constant supply of carrier material and CO₂ for the reaction in the first reactor is desired for the continuous production. Similarly, a constant supply of carbon dioxide releasing carrier material into the second reactor is desired. The continuous production of production of pressurized carbon dioxide means that pressurized carbon dioxide (or separated pressurized carbon dioxide) is produced that can be stored or used as explained below. Thus, pressurized carbon dioxide (or separated pressurized carbon dioxide) should not be exposed to the environment.

The reaction of the carrier material occurs in a first reactor and the product, i.e. the carbon dioxide releasing carrier material, is then transferred (e.g. by using a pump) to a second reactor to release the carbon dioxide from the carbon dioxide releasing carrier material.

The transfer of the carbon dioxide releasing carrier material can be done by means of a pump. The pump or any other means for the transfer is generally used to increase the pressure of the carbon dioxide releasing carrier material to the level of the pressure in the second reactor.

The transfer according to method step (b) can also mean that the first reactor mentioned in method step (a) is located at a first location and the second reactor mentioned in method step (c) is located at a second location and the carbon dioxide releasing carrier material is transported by a transporting vehicle, such as a tanker or ship, to the second reactor mentioned in method step (c) that is located at the aforementioned different location. The carbon dioxide releasing carrier material can then pumped to the second reactor at the desired pressure as mentioned below. Then, the obtained separated carrier material, such as hydrogen mentioned in method step (d), can supplied and transported by a transporting vehicle, such as a tanker or ship, to the first reactor mentioned in step (a). Accordingly, the carrier material, such as hydrogen, can be reused for the reaction step mentioned in method step (a).

In step (c), carbon dioxide is removed from the carbon dioxide releasing carrier material. Accordingly, compressed products comprising pressurized carbon dioxide and the carrier material are obtained in the second reactor. It is preferred that the compressed products are gases and/or supercritical materials. Accordingly, it is particularly preferred that supercritical carbon dioxide is obtained. Particularly, supercritical carbon dioxide and supercritical hydrogen can be obtained. The pressure can be regulated by any means known in the prior art. For instance, the second reactor can have a pressure regulation means such as a pressure valve. Alternatively, the pressure regulation means are arranged within the reactor system, such as implemented within the second or third connecting element. The reactor system and the particular components are described in detail below. The pressure always refers to the absolute pressure unless otherwise specified.

Connecting elements are intended to physically connect specific elements such as the reactors. Connecting elements establish a fluid connection in the reactor system. However, the reactor system can have pressure regulation means present at the in- and outlets of the specific reactors to control the pressure. Moreover, the reactor system such as the connecting elements can comprise valves or other means for controlling the inflow of the substances as required. The connecting elements are preferably tubes or conduits, such as tubular conduits.

The method for the continuous production of pressurized carbon dioxide comprises the following steps (a) reacting, such as reducing, particularly hydrogenating, electrochemically reducing and/or hydrothermal reducing, the carbon dioxide in a first reactor, particularly a pressure regulated first reactor, to obtain a carbon dioxide releasing carrier material, (b) transferring the carbon dioxide releasing carrier material to a second reactor, particularly a pressure regulated second reactor, (c) releasing carbon dioxide from the carbon dioxide releasing carrier material in a second reactor to thereby obtain compressed products comprising pressurized carbon dioxide and the carrier material, (d) separating the compressed products comprising pressurized carbon dioxide and the carrier material obtained in step (c) to obtain pressurized carbon dioxide, and (e) optionally supplying the first reactor with the carrier material, such as the separated carrier material, obtained in step (d) for the reaction, such as reducing, particularly hydrogenating, electrochemically reducing and/or hydrothermal reducing, in step (a). The method can preferably be performed in a reactor system as described below.

This method provides a continuous production of compressed or pressurized carbon dioxide, wherein the energy for obtaining the compressed products is significantly decreased since the method does not need a mechanical compressor for the carbon dioxide. Moreover, the carrier material is separated from the carbon dioxide and can be reused and thus, is not exposed to the environment. Accordingly, the carrier material, such as hydrogen, can be reused in the inventive reactor system or method according to the invention and thus, used in a reaction cycle. Alternatively, the compressed and separated carrier material after the separation can be provided such as collected as mentioned below.

The continuous production with respect to the method or system generally means that the specific components are continuously supplied to the respective reactor or the separator. For instance, the transfer step (b) generally means that the carbon dioxide releasing carrier material is continuously supplied into the second reactor. The same holds true for the carrier material that is continuously supplied into the first reactor.

The carrier material can be any suitable material that is able to reversible receive carbon dioxide. Reversible means that the carbon dioxide releasing carrier material is able to release the carbon dioxide in the releasing step (c), wherein the initial carrier material is obtained. It is particularly preferred that the carbon dioxide releasing carrier material is liquid under moderate conditions, such as conditions as specified for the second reactor or for the carbon dioxide releasing carrier material. In other words, liquid carbon dioxide releasing carrier material is transferred into the second reactor and is also present in the second reactor as a liquid. The liquid carbon dioxide releasing carrier material releases carbon dioxide and the carrier material and it is desired that the released carbon dioxide and carrier material are gases or supercritical materials so that the pressure can be maintained/adjusted/increased in the second reactor for the continuous production of pressurized carbon dioxide.

For instance, the liquid carbon dioxide releasing carrier material is supplied/transferred to the second reactor. Accordingly, means to transfer said liquid carbon dioxide releasing carrier material only has to transfer a liquid material.

The carrier material is preferably hydrogen (H₂). Depending on the used carrier material, the carbon dioxide releasing carrier material can be formic acid, formic acid salts (formates), formaldehyde, amine stabilized formic acid, amine stabilized formaldehyde, methanol, alcohols, carboxylic acids, aldehydes, and/or alkyl formates, preferably formic acid.

Particularly, the carrier material is H₂ and the carbon dioxide releasing carrier material is formic acid, formic acid salts (formates), formaldehyde, amine stabilized formic acid, amine stabilized formaldehyde, methanol, alcohols, carboxylic acids, aldehydes, and/or alkyl formates, preferably formic acid.

The release in step (c) can be a dehydrogenation of the carbon dioxide releasing carrier material in a second reactor to thereby obtain compressed products comprising pressurized carbon dioxide and the carrier material, wherein the carrier material is hydrogen.

It is particularly preferred that the CO₂ is reduced using H₂ as the carrier material to obtain formic acid (HCOOH) as the carbon dioxide releasing carrier material. H₂, such as low pressure H₂ can be directly supplied to the first reactor or generated in the first reactor.

As mentioned above, step (a) in the above-mentioned method requires the reaction, such as reduction, of carbon dioxide using the carrier material in a first reactor to obtain a carbon dioxide releasing carrier material. In other words, the reaction between carbon dioxide and the carrier material, such as hydrogen. Reduction can include hydrogenating, electrochemically reducing and/or hydrothermal reducing. Generally, all possible means to obtain the carbon dioxide releasing carrier material can be used.

If hydrogen is used as carrier material and thus, carbon dioxide releasing carrier material is formic acid, the reaction in step (a) will be the hydrogenation of the carbon dioxide using hydrogen to obtain the carbon dioxide releasing carrier material and step (c) will be the dehydrogenation of the carbon dioxide releasing carrier material in a second reactor to thereby obtain compressed products comprising pressurized carbon dioxide and the carrier material.

For the reaction, such as reduction, it is possible to provide hydrogen (H₂) to the first reactor so that carbon dioxide can be reduced to obtain the carbon dioxide releasing carrier material. The H₂ that is used for the reduction of the carbon dioxide can refer to low pressure hydrogen or non-compressed hydrogen. Low pressure means that the pressure of the aforementioned hydrogen is lower than the pressure of pressurized hydrogen that can be obtained according to the invention. However, it is also possible that the hydrogen is generated *in-situ* in the first reactor. Accordingly, a material can be provided to the first reactor that is able to generate H₂. For instance, H₂O can be used in the electrochemically reducing step to provide the carbon dioxide releasing carrier material. Low pressure hydrogen can refer to a hydrogen that has a pressure of 1 to 100 bar, preferably 1.1 bar to 80 bar and/or the pressure is lower than the pressure of the obtained pressurized hydrogen. Nevertheless, it is beneficial that the pressure of the hydrogen, such as the low-pressure hydrogen, that is supplied to the first reactor, has the same pressure as the pressure in the first reactor.

It is desired that the hydrogen, such as low-pressure hydrogen, is supplied to the first reactor continuously. Step (e) of the inventive method comprise that carrier material, such as H₂, can be supplied to the first reactor. In this case, the hydrogen circulates in the method and/or reactor system. Accordingly, it is desired that the pressure of the carrier material should be adapted to the pressure in the first reactor.

For the reaction, such as reduction, it is possible to provide carbon dioxide (CO₂) to the first reactor so that carbon dioxide can be reduced using hydrogen to obtain the carbon dioxide releasing carrier material. The CO₂ that is used for the reaction can refer to low pressure CO₂ or non-compressed CO₂. Low pressure means that the pressure of the aforementioned CO₂ is lower than the pressure of pressurized CO₂ that can be obtained according to the invention. However, it is also possible that the CO₂ is generated *in-situ* in the first reactor. Accordingly, a material can be provided to the first reactor that is able to generate CO₂. Low pressure CO₂ can refer to CO₂ that has a pressure of 1 to 100 bar, preferably 1.1 bar to 80 bar and/or the pressure is lower than the pressure of the obtained pressurized CO₂. It is desired that the CO₂, such as low-pressure CO₂, is supplied to the first reactor continuously. Nevertheless, it is beneficial that the pressure of the CO₂, such as the low-pressure CO₂, that is supplied to the first reactor, has the same pressure as the pressure in the first reactor.

The electrochemically reduction in method step (a) is carried out using CO₂ and H₂O and/or H₂ as a carrier material to obtain formic acid, methanol, or alcohols as the carbon dioxide releasing carrier material, preferably the electrochemically reducing in step (a) is carried out using CO₂ and H₂O to obtain formic acid as the carbon dioxide releasing carrier material.

The reduction of the carrier material can be done under hydrothermal conditions with a zero-valent metal (M°) that produces formic acid. Water is normally used as the sole source of hydrogen as carrier material for the reduction of CO₂. Preferably, an organic compound such as glycerin is used to regenerate the metal, wherein the glycerin is oxidized to lactic acid that should be constantly removed from the process. Accordingly, in the hydrothermal reduction process of carbon dioxide, H₂O as well as glycerin can be supplied to the first reactor.

The molar ratio of the carrier material, such as hydrogen, to carbon dioxide can be 1/10 to 10/1, such as 1/1 to 10/1, 1/1 to 1/10, 2/1 to 1/2, 1/1 to 3/1, or 1/1 to 1/3.

The carrier material obtained in method step (c) (i.e. separated carrier material) should be supplied to the first reactor. However, it is also possible to supply further carrier material to the first reactor, or to the first reactor *via* a second connecting element. The supply of further carrier material can be beneficial for a continuous method so that a constant level of carrier material is present in the reactor system. However, most of the carrier material that is used for the reaction, such as reduction, in method step (a) should be obtained in the releasing step. Accordingly, the carrier material is substantially the same or the same during the continuous production of the pressurized carbon dioxide. Preferably, more than 60 wt.-%, such as 70 wt.-%, 80 wt.-%, 90 wt.-%, 95 wt.-%, 99 wt.-% or 100 wt.-%, of the carrier material used in method step (a) is obtained in method step (d) and the remaining carrier material along with further carrier material is supplied into the first reactor. Thus, the carrier material should be recycled in the continuous production of the pressurized carbon dioxide. However, if the carrier material is collected as pressurized carrier material, such as pressurized hydrogen (H₂), carrier material should be or has to be supplied to the first reactor so that the continuous production of the carbon dioxide is possible.

The reaction, such as reduction, in method step (a) can be carried out using a catalyst, preferably a ruthenium catalyst, palladium catalyst, molybdenum catalyst and/or iridium catalyst. The ligands of the catalyst are preferably amine-based ligands such as pyridine-based ligands, imidazole-based ligands, 2-imidazoline-based ligands or pyrimidine-based ligands, and/or phosphorus-based ligands such as phosphine ligands, or combinations thereof. The reaction, such as reduction, particularly hydrogenation, electrochemically reduction and/or hydrothermal reduction, in method step (a) is preferably carried out using heterogenous and/or homogenous catalyst, preferably a fixed-bed catalyst.

The specific conditions in the first reactor can be chosen as desired. However, it is preferred that the pressure in the first reactor for the reaction, such as reduction, is 1 to 1000 bar, such as 1 to 100 bar, 1 to 50 bar, 5 to 50, 10 to 40 bar or 20 to 40 bar. The pressure is preferably chosen so that most of the carbon dioxide is reduced to carbon dioxide releasing carrier material. The pressure of the first rector can be controlled by pressure control means and/or a pressure control outlet, such as a pressure valve outlet. Preferably, the pressure in the first reactor is lower than the pressure in the second reactor.

The reaction, such as reduction (e.g. hydrogenating of the carbon dioxide) and/or releasing (e.g. dehydrogenation) (first and/or second reactor, respectively) can require the supply of energy, depending on whether the reaction (reduction, hydrogenating) and/or releasing (e.g. dehydrogenation) is an endothermic or exothermic reaction. Energy can be supplied by heating the first and/or second reactor, particularly the substances and materials inside the first and/or second reactor. On the other hand, heat can be removed from the first or second reactor by a heat exchanger. The temperature in the first reactor for the reaction (e.g. reduction, such as hydrogenating) can be 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C. The reaction (e.g. reduction, particularly hydrogenation, electrochemically reduction and/or hydrothermal reduction), of the carbon dioxide is generally exothermic so that heat is obtained. Accordingly, during the reaction of the carbon dioxide the heat can be dispatched using a heat exchanger so that the temperature in the first reactor can be 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C.

It is desired that the carrier material for the reaction (e.g. reduction, particularly hydrogenation, electrochemically reduction and/or hydrothermal reduction) in method step (a) is dissolved in a solvent. The solvent is preferably ionic liquids, water, 1,4-dioxane, dimethyl sulfoxide (DMSO), alcohols such as methanol and/or N,N-dimethylformamide (DMF).

It is further desired that the solvent comprise a base, such as NEt₃, 1,8-diazabicyclo[5.4.0]undec-7-ene (DBU), KOH, KHCO₃, HCO₂Na, NHex₃ and/or triacetonamines, preferably 2,2,6,6-Tetramethyl-4-piperidone (TAA), n-butyl triacetonediamine (n-Bu TAD), 4-amino-2,2,6,6-tetramethylpiperidine (TAD), N,N-dimethyl-N'-(2,2,6,6-tetramethylpiperidin-4-yl)propane-1,3-diamine (TAT), 4-dimethylamino-2,2,6,6-tetramethylpiperidine (DMA-TEMP), N,N'-bis(2,2,6,6-tetramethylpiperidin-4-yl)hexane-1,6-diamine (HMBTAD). The base is beneficial for the reaction step as well as the release step.

It is preferred that the carrier material is present in the solvent (i.e. dissolved in a solvent) in an amount of 1 to 90 wt.-%, such as 1 to 80 wt.-%, 10 to 80 wt.-% 15 to 50 wt.-%, or 20 to 40 wt.-%, based on the total weight of the solvent mixture. It is preferred that the base is present in the solvent (i.e. dissolved in a solvent) in an amount of 0.05 to 5 wt.-%, such as 0.1 to 4 wt.-%, 0.5 to 3 wt.-% 1 to 5 wt.-%, or 1 to 2 wt.-%, based on the total weight of the solvent mixture. These amounts can refer to the mixture in the first reactor and/or the mixture in the first connecting element.

It is further possible that buffer tanks for the carrier material obtained in method step (d), for further carrier material, and/or for the non-pressurized (or low pressure) carbon dioxide are present. Buffer tanks are useful since the continuous production of the pressurized carbon dioxide can be achieved even during production fluctuations. Moreover, it is possible to maintain certain parts of the reactor system, while the remaining part of the reactor system can operate unhindered.

Method step (a) can further include the purification or concentration of the carbon dioxide releasing carrier material, particularly before transferring the carbon dioxide releasing carrier material into the second reactor and particularly preferred before entering the pump. This is particularly useful so that no contamination of undesirable material is transferred to the second reactor that can negatively affect the release of the carbon dioxide releasing carrier material. Additionally, the purification or concentration of the carbon dioxide releasing carrier material reduces the amount that is transported or transferred, preferably via a pump, to the second reactor. Accordingly, a smaller amount of energy is required for the transfer of the carbon dioxide releasing carrier material into the second reactor.

After the reaction of the carbon dioxide, the carbon dioxide releasing carrier material is transferred to the second reactor. The transfer can be done by any means known in the art such as using a pump. If the carbon dioxide releasing carrier material is dissolved in a solvent, transfer comprises the transfer of the carbon dioxide releasing carrier material dissolved in a solvent, i.e. the carbon dioxide releasing carrier material as well as the solvent. The same holds true if a base is present. However, it is desired that the catalyst remains in the first reactor. Preferably, the catalyst, and major amount such as 50 to 90 wt.-% of solvent and base remains in or is recycled back to the first reactor.

It is particularly desired that the carbon dioxide releasing carrier material is a fluid such as a liquid or gas, preferably the carbon dioxide releasing carrier material is a liquid. In other words, it is particularly desired that the carbon dioxide releasing carrier material is a liquid under the conditions used in the method or reactor system according to the invention. Most preferably, the carbon dioxide releasing carrier material is liquid under the conditions present in the second reactor.

A liquid is generally substantially non-compressible or non-compressible. This means that a substance is not able of being compressed or compressed to a negligible amount. Thus, a non-compressible liquid has a constant volume.

If the carbon dioxide releasing carrier material is a liquid, a pump will merely transfer a considerably low volume into the second reactor compared to gaseous carbon dioxide, wherein the second reactor has preferably a higher pressure level compared to the first reactor. Accordingly, the required energy for transferring the carbon dioxide releasing carrier material instead of gaseous carbon dioxide is considerably lower. Moreover, nearly no pressure-volume work is required. Thus, the energy consumption is significantly lower and a cooling device is generally not necessary but can be present if desired.

The transfer of the carbon dioxide releasing carrier material to the second reactor generally provides the educts to the second reactor, where the release (e.g. dehydrogenation) occurs.

The method step of transferring the carbon dioxide releasing carrier material (b) can include (b1) increasing the pressure of the carbon dioxide releasing carrier material so that the carbon dioxide releasing carrier material is supplied to the second reactor at a pressure of 10 to 4000 bar, such as 50 to 2000 bar, 100 to 1000 bar, 400 to 1000 bar or 600 to 900 bar, (b2) increasing the pressure of the carbon dioxide releasing carrier material to a pressure used in the second reactor for the releasing (e.g. dehydrogenation) in method step (c), and/or (b3) receiving the carbon dioxide releasing carrier material from the first reactor having a first pressure, preferably 1 to 100 bar, such as 1 to 50 bar, 5 to 50, 10 to 40 bar or 20 to 40 bar, and increasing the pressure of the carbon dioxide releasing carrier material to a second pressure, preferably 10 to 4000 bar, such as 50 to 2000 bar, 100 to 1000 bar, 400 to 1000 bar or 600 to 900 bar, wherein the second pressure is higher than the first pressure, wherein preferably a pump is used in options (b1), (b2) and/or (b3) to increases the pressure.

The transfer of method step (b) can further include the supply with further carbon dioxide releasing carrier material into the second reactor.

In the releasing (e.g. dehydrogenation) step, compressed products comprising pressurized carbon dioxide and the carrier material are obtained. Consequently, the release (e.g. dehydrogenating) (c) can be carried out under pressure. The release (e.g. dehydrogenation) is preferably carried out at a pressure of 10 to 4000 bar, such as 50 to 2000 bar, 74 to 1000 bar, 100 to 1000 bar, 400 to 1000 bar or 600 to 900 bar. It is preferred that the pressure in the second reactor is higher than the pressure in the first reactor. The pressure is preferably selected depending on the desired pressure of the pressurized carbon dioxide. Generally, high pressure is desired for the releasing (e.g. dehydrogenation) of the carbon dioxide releasing carrier material in order to obtain pressurized carbon dioxide. The pressure of the second reactor can be controlled by pressure control means and/or a pressure control outlet for the compressed products and/or the pressurized carbon dioxide, such as a pressure valve outlet.

The reaction (e.g. reduction, particularly hydrogenation, electrochemically reduction and/or hydrothermal reduction) and/or releasing (e.g. dehydrogenation) (first and/or second reactor, respectively) can require the supply of energy, depending on whether the reaction (e.g. reduction, particularly hydrogenation, electrochemically reduction and/or hydrothermal reduction) and/or releasing (e.g. dehydrogenation) is an endothermic or exothermic reaction. Energy can be supplied by heating the first and/or second reactor, particularly the substances and materials inside the first and/or second reactor. On the other hand, heat can be removed from the first or second reactor by a heat exchanger. The temperature in the second reactor for the release (e.g. dehydrogenation) can be 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C. The release (e.g. dehydrogenation) of carbon dioxide from the carbon dioxide releasing carrier material is generally endothermic so that additional heat is required for the chemical reaction. Accordingly, during the release (e.g. dehydrogenation) of carbon dioxide from the carbon dioxide releasing carrier material the heat can be supplied so that the temperature in the second reactor can be 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C. It is also possible that the heat of the exothermic reaction is used to supply heat to the endothermic reaction. This is particularly desired to save energy and provide an environmentally friendly continuous process.

As mentioned above, the compressed products are obtained during the release (e.g. dehydrogenation) method step (c) in the second reactor. The compression can be achieved by increasing the pressure of carbon dioxide releasing carrier material in method step (b), particularly by compression means such as a pump. This means that the carbon dioxide releasing carrier material can be transferred to the second reactor by a pump and thus, a pressure difference between the first reactor, (and/or the second connecting element) and the second reactor can be overcome. It is desired that the pressure in the second reactor is higher than the pressure in the first reactor during the continuous production of pressurized carbon dioxide. The transfer of the carbon dioxide releasing carrier material to the second reactor can be achieved by any means known in the art.

It is desired that the compression is achieved by the release (e.g. dehydrogenation) of carbon dioxide (and the carrier material such as H₂) from the carbon dioxide releasing carrier material in method step (c). Thus, after the transfer of the carbon dioxide releasing carrier material to the second reactor and during the release (e.g. dehydrogenation), compressed products comprising pressurized carbon dioxide and the carrier material are obtained in the second reactor. Thus, the compression can be achieved and/or maintained by releasing carbon dioxide gas (or supercritical carbon dioxide) from a carbon dioxide releasing carrier material, such as a liquid carbon dioxide releasing carrier material and/or a substantially non-compressible carbon dioxide releasing carrier material, in a second reactor (preferably pressure regulated second reactor) during the release (e.g. dehydrogenation) of the carbon dioxide from the carbon dioxide releasing carrier material. Moreover, the compression can be achieved and/or maintained by the dehydrogenation of the carbon dioxide releasing carrier material to obtain hydrogen (i.e. carrier material) as well as carbon dioxide. The released gases can be supercritical gases. For the continuous production of pressurized carbon dioxide, it is desired that the carbon dioxide releasing carrier material is continuously supplied to the second reactor.

In a continuous process, the pressure should be constant in the reactors, particularly in the second reactor, where the release occurs. However, the compressed products comprising the pressurized carbon dioxide and the carrier material obtained in method step (c) are separated (i.e. separated pressurized carbon dioxide and separated carrier material). Pressurized carbon dioxide can be obtained and thus, removed from the reactor system. In order to provide a constant pressure in the second reactor, the compression is maintained or can be achieved by releasing carbon dioxide gas (or supercritical CO₂) from a carbon dioxide releasing carrier material as described above (release/dehydrogenation step). The reactor system, particularly the first and/or second reactor, can comprise means to regulate the pressure in the respective reactors.

The release in method step (c) and/or the transfer in method step (b) should be the only means for the compression of the pressurized carbon dioxide. In other words, the release in method step (c) and/or the transfer in method step (b) should be the only means to receive compressed products and thus pressurized carbon dioxide. It is desired that the pressurized carbon dioxide is not compressed by a mechanical compressor.

The release (e.g. dehydrogenation) in method step (c) can be carried out using a catalyst, preferably a ruthenium catalyst, palladium catalyst, molybdenum catalyst and/or iridium catalyst, wherein preferably the ligands of the catalyst are amine-based ligands such as pyridine-based ligands, imidazole-based ligands, 2-imidazoline-based ligands or pyrimidine-based ligands, and/or phosphorus-based ligands such as phosphine ligands, or combinations thereof. The release in method step (c) is preferably carried out using heterogenous and/or homogenous catalyst, preferably a fixed-bed catalyst.

As mentioned before, a solvent can be used in the method according to the invention so that the carbon dioxide releasing carrier material can be transferred into the second reactor with the solvent and an optional base. Thus, the release in method step (c) is carried out in a solvent. In this case the transfer in method step (b) includes the solvent, and optionally the base, in which the carbon dioxide releasing carrier material is dissolved.

The solvent can be continuously resupplied to the first reactor so that the concentration of the carbon dioxide releasing carrier material in the second reactor and/or first reactor is substantially the same.

Alternatively, part of the solvent or the entire solvent is separated before entering the second reactor and resupplied to the first reactor. Thus, the method step (b) and/or (c) can further include that solvent (including an optional base), such as 1 to 100 wt.%, 10 to 99 wt.%, 50 to 95 wt.%, 80 to 90 wt.%, 10 to 50 wt.%, 10 to 20 wt.%, or 30 to 50 wt.% of the solvent, is separated and returned to the first reactor. The wt.-% is based on the total weight of the solvent (including an optional base) present in the solved carbon dioxide releasing carrier material before the separation. Particularly, solvent is separated and returned to the first reactor before entering the second reactor, preferably before entering the pump and/or the solvent is separated and returned from the second reactor to the first reactor. It is desired that the separation of the solvent (optionally including a base) occurs before the transfer step. In other words, the separation occurs before the carbon dioxide releasing carrier material enters the pump for supplying the carbon dioxide releasing carrier material to the second reactor. However, even if solvent is not transferred into the second reactor, the release (c) can be carried out in a solvent as mentioned above that simply remains in the second reactor.

It is desired that the carbon dioxide releasing carrier material for the dehydrogenation in method step (c) is dissolved in a solvent. The solvent is preferably water, ionic liquids, 1,4-dioxane, dimethyl sulfoxide (DMSO), alcohols such as methanol and/or N,N-dimethylformamide (DMF).

It is further desired that the solvent comprise a base, such as NEt₃, 1,8-diazabicyclo[5.4.0]undec-7-ene (DBU), KOH, KHCO₃, HCO₂Na, NHex₃ and/or triacetonamines, preferably 2,2,6,6-Tetramethyl-4-piperidone (TAA), n-butyl triacetonediamine (n-Bu TAD), 4-amino-2,2,6,6-tetramethylpiperidine (TAD), N,N-dimethyl-N'-(2,2,6,6-tetramethylpiperidin-4-yl)propane-1,3-diamine (TAT), 4-dimethylamino-2,2,6,6-tetramethylpiperidine (DMA-TEMP), N,N'-bis(2,2,6,6-tetramethylpiperidin-4-yl)hexane-1,6-diamine (HMBTAD). The base is beneficial for the dehydrogenation step.

It is preferred that the carbon dioxide releasing carrier material is present in the solvent (i.e. dissolved in a solvent) in an amount of 1 to 90 wt.-%, such as 1 to 80 wt.-%, 10 to 80 wt.-% 15 to 50 wt.-%, or 20 to 40 wt.-%, based on the total weight of the solvent mixture. It is preferred that the base is present in the solvent (i.e. dissolved in a solvent) in an amount of 0.05 to 2 wt.-%, such as 0.1 to 4 wt.-%, 0.5 to 3 wt.-% 1 to 5 wt.-%, or 1 to 2 wt.-%, based on the total weight of the solvent mixture. These amounts can refer to the mixture in the second reactor.

Compressed products comprising pressurized carbon dioxide and the carrier material can be transferred via a connecting element between the second reactor and the separator to a separating step (d). Then the compressed products are separated to obtain the separated pressurized carbon dioxide and the separated carrier material. Since compressed products are obtained, the separated carrier material (or carrier material) is also pressurized. It is also possible that the carbon dioxide is separated as a solid product.

The separation in method step (d) can include the condensation of the CO₂, such as the condensation of supercritical CO₂. Accordingly, the carrier material and the pressurized carbon dioxide are separated. However, other methods for separating the compressed products are also suitable such as using an alkaline gas scrubber, amine gas treating, a separator using a membrane and/or other separation mechanism (e.g. electrochemical).

The method can further include the step (f1) collecting the pressurized carbon dioxide (i.e. the separated and pressurized CO₂), such as in pressure vessels for storage or transportation, (f2) using the pressurized carbon dioxide (i.e. the separated and pressurized CO₂) for chemical reactions and/or (f3) feed the pressurized carbon dioxide (i.e. the separated and pressurized CO₂) into carbon dioxide pipelines or carbon dioxide supply networks e.g. for transportation or storage of carbon dioxide into underground or sub-sea level CO₂ deposits (i.e. CCS). Said pressurized carbon dioxide refers to the separated pressurized carbon dioxide. Moreover, it is also desirable to (f4) collecting the separated and compressed carrier material, such as H₂, preferably in pressure tanks, (f5) using the separated and compressed carrier material, such as H₂, for chemical reactions, and/or (f6) feed the separated and compressed carrier material, such as H₂, into hydrogen pipelines or hydrogen supply networks. Said pressurized hydrogen refers to the separated pressurized hydrogen. Accordingly, it is possible to resupply the carrier material after the separation to the first reactor, collect/use said carrier material or both.

The pressure of the pressurized carbon dioxide, i.e. the separated pressurized carbon dioxide, can be 10 to 4000 bar, such as 50 to 2000 bar, 74 to 1000 bar, 100 to 1000 bar, 400 to 1000 bar or 600 to 900 bar. The pressure of the pressurized hydrogen (as carrier material), i.e. the separated pressurized hydrogen, can be 10 to 4000 bar, such as 50 to 2000 bar, 74 to 1000 bar, 100 to 1000 bar, 400 to 1000 bar or 600 to 900 bar. However, it is desirable that the pressure of the pressurized carbon dioxide is the same or lower than the pressure in the second reactor. The separated pressurized carbon dioxide is preferably not mechanically compressed before or after (f1) collecting the pressurized carbon dioxide, (f2) using the pressurized carbon dioxide for chemical reactions and/or (f3) feed the pressurized carbon dioxide into carbon dioxide pipelines or carbon dioxide supply networks.

Furthermore, it is desired that the pressurized carbon dioxide, i.e. the separated pressurized carbon dioxide, is purified in order to remove undesired by-products such as CO and residual H₂. However, it is possible that CO is formed during the release step (e.g. dehydrogenation). Accordingly, the separated pressurized carbon dioxide can comprise less than 5 wt.-% CO, such as less than 0.01 wt.-%, less than 0.001 wt.-%, 0.0005 to 0.001 wt.-%, or 0.0001 to 0.001 wt.-%, based on the total weight of the separated pressurized carbon dioxide. Moreover, the separated pressurized carbon dioxide can comprise less than 10 wt.-% CO₂, such as less than 1 wt.-%, less than 0.1 wt.-%, 0.05 to 5 wt.-%, 0.1 to 4 wt.-%, 0.2 to 2 wt.-%, or 0.5 to 1 wt.-%, based on the total weight of the separated pressurized carbon dioxide.The separated pressurized hydrogen can comprise less than 5 wt.-% CO, such as less than 0.01 wt.-%, less than 0.001 wt.-%, 0.0005 to 0.001 wt.-%, or 0.0001 to 0.001 wt.-%, based on the total weight of the separated pressurized hydrogen. Moreover, the separated pressurized hydrogen can comprise less than 10 wt.-% CO₂, such as less than 1 wt.-%, less than 0.1 wt.-%, 0.05 to 5 wt.-%, 0.1 to 4 wt.-%, 0.2 to 2 wt.-%, or 0.5 to 1 wt.-%, based on the total weight of the separated pressurized hydrogen. Accordingly, the separation of the compressed products should completely separate the compressed products or the pressurized carbon dioxide and the carrier material (such as hydrogen) but it is possible that the compressed products are not completely separated.

Thus, the method can further comprise (h) purifying the pressurized carbon dioxide separated in step (d) using a membrane, an alkaline gas scrubber, a condenser, amine gas treating and/or electrochemical separation. The amine gas treating can be done as disclosed in US 2015/0125373 A1 using an absorption medium containing a 4-amino-2.2.6.6-tetramethylpiperidine substituted on the 4-amino group with the substituent on the 4-amino group carrying a suitable functional group which improves water solubility. The absorption medium is shown in paragraph 11 ff. of US 2015/0125373 A1. Accordingly, the absorption medium can be a triacetonamine and its derivates.

In method step (e), the first reactor can be supplied with the carrier material obtained in method step (d) for the reaction (e.g. reduction, particularly hydrogenation, electrochemically reduction and/or hydrothermal reduction), in method step (a).

The supply in method step (e) can further include the adjustment of the pressure of the carrier material to the pressure in the first reactor such as 1 to 1000 bar, such as 1 to 100 bar, 1 to 50 bar, 5 to 50, 10 to 40 bar or 20 to 40 bar, preferably using a device for pressure control, particularly a pressure control outlet such as pressure control valve, a device for energy recovery such as an expander/turbine and/or a heat exchanger, and/or a flash evaporation chamber.

The pressure of the separated pressurized carbon dioxide can be adjusted to 10 to 4000 bar, such as 50 to 2000 bar, 74 to 1000 bar, 100 to 1000 bar, 400 to 1000 bar or 600 to 900 bar, preferably using a device for pressure control, particularly a pressure control outlet such as pressure control valve, a device for energy recovery such as an expander/turbine and/or a heat exchanger, and/or a flash evaporation chamber. However, it is desired that the adjusted pressure of the separated pressurized carbon dioxide is equal or lower than the pressure in the second reactor. The obtained separated pressurized carbon dioxide can be present in a gaseous, supercritical, liquid or solid state.

The carrier material (e.g. hydrogen), such as the separated carrier material, can be heated and/or cooled after the adjustment of the pressure, preferably to 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C, more preferably to a temperature used for the reaction (e.g. reduction, particularly hydrogenation, electrochemically reduction and/or hydrothermal reduction), in method step (a) in the first reactor.

It is also possible to heat or cool the pressurized carbon dioxide after the adjustment of the pressure, preferably to 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C.

The present invention is also directed to a reactor system for the continuous production of pressurized carbon dioxide, preferably using the method as defined above. The reactor system for the continuous production of pressurized carbon dioxide comprises (a) a first reactor, particularly a pressure regulated first reactor, for the reaction, such as reduction, particularly hydrogenation, electrochemically reduction and/or hydrothermal reduction, of carbon dioxide to obtain a carbon dioxide releasing carrier material, (b) a first connecting element for the transfer of the carbon dioxide releasing carrier material from the first reactor to a second reactor, (c) a second reactor, particularly a pressure regulated second reactor, for the releasing of the carbon dioxide from the carbon dioxide releasing carrier material to thereby obtain compressed products comprising pressurized carbon dioxide and the carrier material, (d) a separator in connection with the second reactor (c) for the separation of the compressed products comprising the pressurized carbon dioxide and the carrier material, (e) optionally a second connecting element connected to the separator for the transfer of the carrier material from the separator (d) to the first reactor (a).

It is particularly preferred that the reactor system does not comprise a pressure pump (or a mechanical compressor) for the compression of the pressurized carbon dioxide and/or carbon dioxide. A pump can be used for the transfer of the carbon dioxide releasing carrier material but not for the compression of the pressurized carbon dioxide and/or carbon dioxide.

The reactor system can further comprise a third connecting element for the transfer of a solvent from the second reactor (a) to the first reactor (c). A buffer tank for the solvent can be arranged within the third connecting element.

The reactor system can further comprise a device for pressure control, particularly a pressure control outlet such as pressure control valve, and/or a device for energy recovery such as an expander/turbine and/or a heat exchanger, a pressure control valve and/or a flash evaporation chamber, preferably located between the separator (d) and the first reactor (a) and arranged within the second connecting element (e), arranged between the separator (d) and the second reactor (c), and/or connected to the separator (d) for the separated the pressurized carbon dioxide.

It is preferred that the first reactor (a) and the second reactor (c) are directly connected by the first connecting element (c) and/or the second connecting element (e). Directly connected means that the first and the second reactor are physically connected with each other. Directly does not exclude further components that are present within the respective connecting element such as a pump.

The connecting elements are preferable tubes, or conduits, such as tubular conduits, that can comprise various components such as a valve, buffer tank, injectors, separators, heat exchangers, or measurement instruments.

The volume of the first reactor (a) can be 100 mL to 2.000.000 L, such as 1 L to 200.000 L, 100 L to 100.000 L or 1.000 L to 50.000 L and can have a cylindrical shape. It is also possible that several first reactors can operate in series or side by side so that the second connecting element is able to connect every first reactor. The first reactor can comprise means for heat exchange, that is preferably connected to means for heat exchange that is present in the second reactor.

Furthermore, the first reactor can comprise means for control the pressure such as a valve. Accordingly, the first reactor can be a pressure regulated first reactor. However, other positions for the pressure regulation can be implemented. For instance, pressure regulation means can be present in the connecting elements as desired.

The reactor system comprises a first connecting element for the transfer of the carbon dioxide releasing carrier material from the first reactor to a second reactor. Thus, the first connecting element physically connects the first and second reactor. The first connecting element as well as every connecting element can be a tube, a conduit or a tubular conduit.

The first connecting element (b) can comprise a pressure pump, such as a radial, axial, piston, and/or membrane pump, to adjust/increase the pressure to the pressure in the second reactor (c). Particularly the pump can adjust/increase the pressure to 10 to 4000 bar, such as 50 to 2000 bar, 100 to 1000 bar, 400 to 1000 bar or 600 to 900 bar.

A buffer tank for the carbon dioxide releasing carrier material can be arranged within the first connecting element. Accordingly, the reactor system can further comprise at least one buffer tank for the carbon dioxide releasing carrier material located between the first reactor (a) and the second reactor (c) and arranged within the first connecting element (b). The buffer tank can regulate the constant supply of carbon dioxide releasing carrier material into the second reactor. The buffer tank can be either arranged before the optional pump or behind the pump with respect to the flow direction of the carbon dioxide releasing carrier material.

The reactor system comprises a second reactor (c), particularly a pressure regulated second reactor, for the release of the carbon dioxide from the carbon dioxide releasing carrier material to thereby obtain compressed products comprising pressurized carbon dioxide and the carrier material.

The volume of the second reactor (c) can be 100 mLto 2.000.000 L, such as 1 Lto 200.000 L, 100 L to 100.000 L or 1.000 L to 50.000 L and can have a cylindrical shape. It is also possible that several second reactors can operate in series or side by side so that the first connecting element is able to connect every second reactor. The second reactor can comprise means for heat exchange, that is preferably connected to means for heat exchange that is present in the first reactor.

The second reactor (c) for the release of the carbon dioxide from the carbon dioxide releasing carrier material and/or the pressure pump that is arranged within the connecting element (b) for the transfer of the carbon dioxide releasing carrier material to a second reactor can be the only means for the compression of the pressurized carbon dioxide and/or to receive compressed products and thus pressurized carbon dioxide.

The second reactor (c) can comprise pressure control means and/or a pressure control outlet for the compressed products and/or the pressurized carbon dioxide, such as a pressure valve outlet, and the separator (d) is connected via the pressure valve outlet with the second reactor (c).

The reactor system comprises a separator (d) in connection with the second reactor (c) for the separation of the compressed products comprising the pressurized carbon dioxide and the carrier material. Accordingly, the separator is able so separate the carrier material and the pressurized carbon dioxide. Accordingly, a separated carrier material and a separated and pressurized carbon dioxide is obtained. It is possible that the separated components comprise impurities. The separator can be connected with the second reactor via a connecting element, such as a tube.

The separator is in connection with the second reactor (c) for the separation of the compressed products comprising the pressurized carbon dioxide and the carrier material. As mentioned above, the second reactor can be directly connected with the first reactor via the second connecting element. This means that the second reactor is in connection with the separator via a connecting element between the second reactor and the separator and the separator is in connection with the first reactor via the second connecting element so that a physical connection is established.

The separator can be any separator that is able to separate the compressed products comprising pressurized carbon dioxide and the carrier material. For example, the separator can be a condenser, particularly a CO₂ condenser, separator using a membrane or an alkaline gas scrubber, amine gas treating and/or other separation mechanism (e.g. electrochemical). The separated carrier material can be then resupplied, such as continuously resupplied, to the first reactor.

A gas-liquid separating method, such as condensing, can be used. The compressed products comprising pressurized carbon dioxide (particularly supercritical carbon dioxide) and the carrier material, such as hydrogen (particularly supercritical hydrogen), can be cooled down to a temperature, where the CO₂, forms a liquid phase and the carrier material, such as hydrogen, forms a gaseous phase. The separated CO₂ can comprise carrier material, such as hydrogen, that was not completely separated from the compressed products. The separated carrier material can comprise CO₂ that was not completely separated from the compressed products. If the separated carrier material, such as H₂ comprises CO₂ after the separation, the separated carrier material comprising remaining CO₂ is transferred to the first reactor. The separated carrier material can comprise less than 5 wt.-% CO, such as less than 0.01 wt.-%, less than 0.001 wt.-%, 0.0005 to 0.001 wt.-%, or 0.0001 to 0.001 wt.-%, based on the total weight of the separated carrier material. Moreover, the separated carrier material can comprise less than 10 wt.-% CO₂, such as less than 5 wt.-%, 1 wt.-%, less than 0.1 wt.-%, 0.05 to 5 wt.-%, 4 to 10 wt.-%, 0.2 to 2 wt.-%, or 0.5 to 1 wt.-%, based on the total weight of the separated carrier material. The separated carbon dioxide can comprise less than 5 wt.-% CO, such as less than 0.01 wt.-%, less than 0.001 wt.-%, 0.0005 to 0.001 wt.-%, or 0.0001 to 0.001 wt.-%, based on the total weight of the separated carbon dioxide. Moreover, the separated carbon dioxide can comprise less than 10 wt.-% hydrogen, such as less than 5 wt.-%, 1 wt.-%, less than 0.1 wt.-%, 0.05 to 5 wt.-%, 4 to 10 wt.-%, 0.2 to 2 wt.-%, or 0.5 to 1 wt.-%, based on the total weight of the separated carbon dioxide.

The required temperature for the condensing of the carrier material depends on the pressure of the compressed products. Compressed products having a pressure of around 300 bar and a temperature of around 80 °C can be separated by cooling the mixture to about -40 °C.

The separated compressed CO₂ can also comprise carrier material, such as H₂ (or further residuals such as CO) that could not be completely separated from hydrogen. Accordingly, the reactor system can further comprise a CO₂ purification chamber for purifying the pressurized carbon dioxide using a membrane, an alkaline gas scrubber, a condenser, electrochemical separation and/or amine gas treating, wherein the carbon dioxide purification chamber is connected to the separator (d) and receives the pressurized carbon dioxide after the separation.

The reactor system can comprise pressure control means or a pressure control outlet for the pressurized carbon dioxide, preferably connected to the separator (d) or a carbon dioxide purification chamber for purifying the pressurized carbon dioxide using a membrane, wherein the carbon dioxide purification chamber is connected to the separator (d) and receives the pressurized carbon dioxide after the separation.

The second connecting element is connected to the separator for the transfer of the carrier material, such as H₂, from the separator (d) to the first reactor (a). A pump can be arranged in the second connecting element to pump the separated carrier material into the first reactor. Alternatively, a pressure regulating valve or expander/turbine can be arranged in the second connecting element to adjust the pressure of the carrier material to the pressure present in the first reactor.

A buffer tank for the carrier material can be arranged within the second connecting element. Accordingly, the reactor system can further comprise at least one buffer tank for the carrier material located between the separator (d) and the first reactor (a) and arranged within the second connecting element (e). The buffer tank can regulate a constant supply of carrier material into the first reactor. The buffer tank can be either arranged before the optional pump or behind the pump with respect to the flow direction of the carrier material.

The reactor system can further comprise a pressure tank, carbon dioxide loading station, carbon dioxide fueling station, pipeline, and/or carbon dioxide supply network for receiving the pressurized carbon dioxide connected to the separator (d). The above-mentioned carbon dioxide purification chamber is arranged in front of the aforementioned elements with respect to the flow direction of the pressurized carbon dioxide.

The present invention is also directed to the use of a carrier material for the continuous production of pressurized carbon dioxide, preferably as defined for the method of the continuous production of pressurized hydrogen and/or preformed in a reactor system as defined before. The use of a carrier material for continuous production of pressurized carbon dioxide includes (a) the release of the carbon dioxide from a carbon dioxide releasing carrier material to thereby obtain compressed products comprising pressurized carbon dioxide and the carrier material, and (b) the reaction, such as reduction, particularly hydrogenation, electrochemically reduction and/or hydrothermal reduction, of the carrier material obtained in the release step (a) to obtain the carbon dioxide releasing carrier material used for the release step (a). If hydrogen is used as carrier material, step (a) can be (a) the dehydrogenation of the carbon dioxide releasing carrier material to thereby obtain compressed products comprising pressurized carbon dioxide and the carrier material that is hydrogen.

The use of a carrier material for the continuous production of pressurized hydrogen means that the carrier material can be reused after the release of the carbon dioxide and not exposed to the environment.

The compression is generally achieved by the release of the carbon dioxide and/or the carrier material, such as hydrogen, (e.g. dehydrogenation) from the carbon dioxide releasing carrier material in step (a), wherein the release (e.g. dehydrogenating) (a) is carried out under pressure, particularly at 10 to 4000 bar, such as 50 to 2000 bar, 100 to 1000 bar, 400 to 1000 bar or 600 to 900 bar.

The pressure should be maintained by the release (e.g. dehydrogenation) of the carbon dioxide from the carbon dioxide releasing carrier material, particularly the pressure is maintained by the release (e.g. dehydrogenation) of the carbon dioxide from the carbon dioxide releasing carrier material, such as the pressure is maintained by releasing the carbon dioxide and/or hydrogen from the carbon dioxide releasing carrier material during the release step.

In other words, the compression should be achieved and/or maintained by releasing carbon dioxide gas (or supercritical carbon dioxide) and/or hydrogen gas (or supercritical H₂) from a carbon dioxide releasing carrier material, such as a liquid carbon dioxide releasing carrier material and/or a substantially non-compressible carbon dioxide releasing carrier material, in a pressure regulated second reactor during the release in step (a) of the carbon dioxide releasing carrier material. Moreover, the carbon dioxide releasing carrier material, preferably liquid carbon dioxide releasing carrier material, should be continuously transferred to the release step in the second reactor.

The invention will now be described with reference to the accompanying figures which do not limit the scope and ambit of the invention. The description provided is purely by way of example and illustration. However, specific features exemplified in the figures can be used to further restrict the scope of the invention and claims.

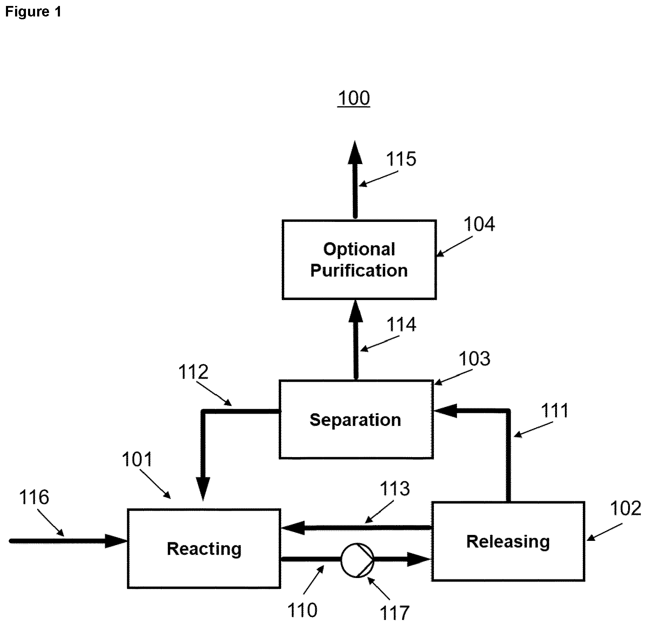

FIG. 1 reveals a flow-chart of the method for the continuous production of pressurized carbon dioxide (100). The method (100) includes reacting, such as reducing, particularly hydrogenating, electrochemically reducing and/or hydrothermal reducing, a carrier material (101). Carbon dioxide and/or a carrier material, such as hydrogen or a hydrogen-generating material, such as water, is supplied (116) to a first reactor for the reaction (101). The carbon dioxide that is provided for the reaction of the carrier material has a lower pressure than the separated pressurized carbon dioxide (115). Accordingly, low pressure carbon dioxide should be provided for the reaction in the first reactor. Low pressure means that the pressure in the first reactor is lower than the pressure in the second reactor.

The reaction (101) can be carried out in a solvent, preferably ionic liquids, water, 1,4-dioxane, dimethyl sulfoxide (DMSO), alcohols such as methanol and/or N,N-dimethylformamide (DMF). Moreover, a base can be added to the mixture to accelerate the reduction of the carrier material. The base can be selected from such as NEt₃, 1,8-diazabicyclo[5.4.0]undec-7-ene (DBU), KOH, KHCO₃, HCO₂Na, NHex₃ and/or triacetonamines, preferably 2,2,6,6-Tetramethyl-4-piperidone (TAA), n-butyl triacetonediamine (n-Bu TAD), 4-amino-2,2,6,6-tetramethylpiperidine (TAD), N,N-dimethyl-N'-(2,2,6,6-tetramethylpiperidin-4-yl)propane-1,3-diamine (TAT), 4-dimethylamino-2,2,6,6-tetramethylpiperidine (DMA-TEMP), N,N'-bis(2,2,6,6-tetramethylpiperidin-4-yl)hexane-1,6-diamine (HMBTAD), or mixtures thereof. The carbon dioxide releasing carrier material can be transferred via a first connecting element (110) using a pump (117) to the second reactor. Transfer of the carbon dioxide releasing carrier material may include the solvent(s) and the base(s). However, it is also possible that the solvent and base is separated from the carbon dioxide releasing carrier material so that only the carbon dioxide releasing carrier material without the solvent and base is transferred to the second reactor (102). Alternatively, the amount of solvent including a possible base can be reduced by at least 99 wt.-%, 95 wt.-%, 90 wt.-%, 80 wt.-%, 70 wt.-%, 50 wt.-%, 10 to 99 wt.-%, 20 to 95 wt.-% 30 to 80 wt.-%, based on the total amount of solvent including a possible base before the separation of the solvent including a possible base and the carbon dioxide releasing carrier material. The amount of solvent including a possible base can be reduced to less than 1 wt.-%, 5 wt.-%, 10 wt.-%, 20 wt.-%, 30 wt.-%, 50 wt.-%, 1 to 90 wt.-%, 5 to 50 wt.-% 10 to 20 wt.-%, of the solvent including a possible base, based on the total weight of the (solved) carbon dioxide releasing carrier material.

Moreover, a catalyst can be used for the reaction of the carrier material (101). The catalyst can be chosen from a ruthenium catalyst, palladium catalyst, molybdenum catalyst and/or iridium catalyst. The ligands of the catalyst are preferably amine-based ligands such as pyridine-based ligands, imidazole-based ligands, 2-imidazoline-based ligands or pyrimidine-based ligands, and/or phosphorus-based ligands such as phosphine ligands, or combinations thereof. For instance, RuH₂(dppm)₂ (dppm=1,1-bis(diphenylphosphino)methane) can be used for the reduction. Another possible iridium catalyst is [Cp*Ir(H₂O)₃](SO₄) + 2-hydrazin-pyridine. The reaction, such as reducing, particularly hydrogenating, electrochemically reducing and/or hydrothermal reducing, can be carried out using heterogenous and/or homogenous catalyst, preferably a fixed-bed catalyst. It is desired that the catalyst remains in the first reactor.

After the transfer of the carbon dioxide releasing carrier material (110) into the second reactor (102), the carbon dioxide releasing carrier material releases carbon dioxide in the second reactor, particularly a pressure regulated second reactor, to thereby obtain compressed products comprising pressurized carbon dioxide and the carrier material, such as hydrogen. The release can be carried out in a solvent, preferably water, ionic liquids, 1,4-dioxane, dimethyl sulfoxide (DMSO), alcohols such as methanol and/or N,N-dimethylformamide (DMF). Moreover, a base can be added to the mixture to accelerate the release of the carbon dioxide. The base can be selected from such as NEt₃, 1,8-diazabicyclo[5.4.0]undec-7-ene (DBU), KOH, KHCO₃, HCO₂Na, NHex₃ and/or triacetonamines, preferably 2,2,6,6-Tetramethyl-4-piperidone (TAA), n-butyl triacetonediamine (n-Bu TAD), 4-amino-2,2,6,6-tetramethylpiperidine (TAD), N,N-dimethyl-N'-(2,2,6,6-tetramethylpiperidin-4-yl)propane-1,3-diamine (TAT), 4-dimethylamino-2,2,6,6-tetramethylpiperidine (DMA-TEMP), N,N'-bis(2,2,6,6-tetramethylpiperidin-4-yl)hexane-1,6-diamine (HMBTAD), or mixtures thereof. Generally, the solvent including the base is separated from the carbon dioxide releasing carrier material and the compressed products, and supplied via a connecting element (113) back to the first reactor (101). However, it is also possible that the solvent remains in the second reactor for the release (e.g. dehydrogenation). Accordingly, it is possible that the first reactor uses a different solvent system than the second reactor and each solvent system remains in the respective reactor.

Moreover, a catalyst can be used for the release of the carbon dioxide (102). The catalyst can be chosen from a ruthenium catalyst, palladium catalyst, molybdenum catalyst and/or iridium catalyst. The ligands of the catalyst preferably are amine-based ligands such as pyridine-based ligands, imidazole-based ligands, 2-imidazoline-based ligands or pyrimidine-based ligands, and/or phosphorus-based ligands such as phosphine ligands, or combinations thereof. For instance, a possible catalyst can be [Cp*Ir(4DHBP)(H₂O)](SO₄), wherein 4DHBP is 4,4'-dihydroxy-2,2'-bipyridine.

The compressed products including the pressurized carbon dioxide and the carrier material are transferred (111) via a connecting element to the separation step (103). The compressed products are generally gaseous (or supercritical materials) so that they can be easily transferred to a separator.

The separation (103) of the carrier material and the pressurized carbon dioxide can be done by any means known in the art. For instance, the temperature can be reduced to less than 30 °C, such as less than 10 °C, 0 °C, -10 °C, -20 °C, -40 °C, or -60 °C, so that the CO₂ becomes liquid and can be easily separated from the gaseous carrier material, such as H₂.

The separated carrier material is resupplied (112) for the reaction (101) into the first reactor. The separated pressurized carbon dioxide can be supplied (114) to an optional purification process (104), where undesired components can be separated. Undesired components can be CO, or H₂ that was not completely removed. Useful purification processes are an alkaline gas scrubber and/or amine gas treating, electrochemical separation, condensation or the purification using a membrane.

The purified pressurized carbon dioxide (115) can be stored in a tank, used for chemical reactions, transportation or distributed into carbon dioxide pipelines or carbon dioxide supply networks. The purified pressurized carbon dioxide (115) can be a liquid, supercritical, gaseous, or solid.

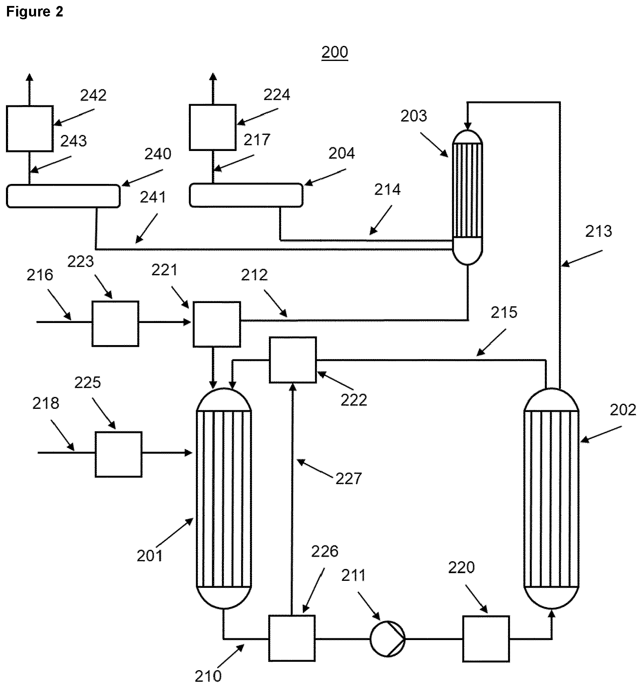

Referring to FIG. 2, the reactor system (200) comprises a first reactor (201) and a second reactor (202). The first reactor (201) and/or the second reactor (202) are preferably pressure regulated. The first and second reactor (201, 202) are physically connected with each other using a connecting element (210). The connecting element (210) can be a pipe or a tubular conduit. In FIG. 2, the connecting element (210) comprises a pump (211), a solvent separator (226) as well as a buffer tank (220). The solvent separator (226) is connected via a connecting element (227) with buffer tank (222) but can alternatively directly connected to the first reactor (201) or to the connecting element (215). The solvent separator (226) is located between the pump (211) and the first reactor (201). The solvent separator (226) is able to separate the solvent including an optional base and the carbon dioxide releasing carrier material. Accordingly, the pump (211) must transfer a significantly lower amount of material to the second reactor (202). The amount of solvent including a possible base can be reduced by at least 99 wt.-%, 95 wt.-%, 90 wt.-%, 80 wt.-%, 70 wt.-%, 50 wt.-%, 10 to 99 wt.-%, 20 to 95 wt.-% 30 to 80 wt.-%, based on the total weight of solvent including a possible base before the transfer of the carbon dioxide releasing carrier material. The amount of solvent including a possible base can be reduced to less than 1 wt.-%, 5 wt.-%, 10 wt.-%, 20 wt.-%, 30 wt.-%, 50 wt.-%, 1 to 90 wt.-%, 5 to 50 wt.-% 10 to 20 wt.-%, of the solvent including a possible base, based on the total weight of the (solved) carbon dioxide releasing carrier material. The solvent separator (226) can completely separate the carbon dioxide releasing carrier material and solvents including an optional base.

The pump (211) is used to transfer the carbon dioxide releasing carrier material to the second reactor (202). As mentioned above, the pressure in the second reactor (202) is higher than the pressure in the first reactor since the compressed products are obtained in the second reactor. It is intended that the pump (211) transfers the carbon dioxide releasing carrier material as well as a solvent (optionally comprising a base) in which the carbon dioxide releasing carrier material is dissolved.

The second reactor (202) is connected with a separator (203) via a connecting element (213). Said connecting element (213) is able to transfer the compressed products to the separator (203).

The separator (203) is connected with the first reactor via a second connecting element (212) so that the separated carrier material can be supplied to the first reactor (201). Accordingly, the reactor system is configured to preferably use the carrier material in a cyclic process but this is not necessary. It is desired that the components or elements of the entire reactor system (200) are in direct fluid communication. The term "direct" means that the reactor system is in physically connection with the described aforementioned elements as shown in FIG. 2 but can also comprise valves or similar components.

The second connecting element (212) comprises a buffer tank (221) so that the carrier material can be constantly supplied to the first reactor (201). If required, further carrier material (216) can be supplied either directly to the first reactor (201) or to the buffer tank (221) as shown in FIG. 2. The connecting element for the further carrier material (216) can also comprise a buffer tank (223).

It is preferred that the carrier material is hydrogen and it is even more preferred that the carrier material is hydrogen and the carbon dioxide releasing carrier material is formic acid or salts thereof. Hydrogen-generating materials can be H₂O or hydrocarbons so that hydrogen as the carrier material can be directly generated, for instance in the first reactor or a reactor upstream to the first reactor.

Carbon dioxide (218) is supplied to the first reactor (201) for the reaction of the carrier material, such as hydrogen, with the carbon dioxide. The carbon dioxide is supplied via a connecting element (218) that comprises a buffer tank (225) (or storage tank) to constantly supply the first reactor with the carbon dioxide.

The separated pressurized carbon dioxide is transferred to an optional purification chamber (204) and thus, the separator (203) and the optional purification chamber (204) are connected with each other via a connecting element (214). After the purification, the purified pressurized carbon dioxide can be stored in a tank (224) and/or distributed via a connecting element (217) into carbon dioxide pipelines or carbon dioxide supply networks (not shown).

It is also desirable to collect the separated hydrogen as carrier material in the inventive method and reactor system. The entire separated hydrogen can be purified and collected or a part of the separated hydrogen can be purified and collected. The remaining part is then resupplied to the first reactor (201). For instance, the separator (203) may comprise two outlets for separated hydrogen as carrier material. It is desired that the portion that has the higher purity after separation is collected and optional purified and the portion that comprises impurities, such as remaining CO₂, is resupplied to the first reactor (201). Thus, the separated pressurized hydrogen can be transferred to an optional purification chamber (240) and thus, the separator (203) and the optional purification chamber (240) are connected with each other via a connecting element (241). After the purification, the purified pressurized hydrogen can be stored in a tank (242) and/or distributed via a connecting element (243) into hydrogen pipelines or hydrogen supply networks (not shown).

It will be appreciated that various modifications can be made, and that many changes can be made in the preferred embodiments without departing from the principle of the invention.

### ASPECTS OF THE INVENTION

1. A method for the continuous production of pressurized carbon dioxide, preferably in a reactor system as defined in aspects 30 to 45, preferably according to the use as defined in aspects 46 to 49, comprising:
   (a) reacting, such as reducing, particularly hydrogenating, electrochemically reducing and/or hydrothermal reducing, carbon dioxide in a first reactor, particularly a pressure regulated first reactor, to obtain a carbon dioxide releasing carrier material,
   (b) transferring the carbon dioxide releasing carrier material to a second reactor, particularly a pressure regulated second reactor,
   (c) releasing carbon dioxide from the carbon dioxide releasing carrier material in a second reactor to thereby obtain compressed products comprising pressurized carbon dioxide and the carrier material,
   (d) separating the compressed products comprising pressurized carbon dioxide and the carrier material obtained in step (c) to obtain pressurized carbon dioxide, and
   (e) optionally supplying the first reactor with the carrier material, such as the separated carrier material, obtained in step (d) for the reaction, such as reducing, particularly hydrogenating, electrochemically reducing and/or hydrothermal reducing, in step (a).
2. A method for the continuous production of pressurized carbon dioxide, preferably in a reactor system as defined in aspects 30 to 45, preferably according to the use as defined in aspects 46 to 49, comprising:
   (a) reducing, particularly hydrogenating, electrochemically reducing and/or hydrothermal reducing, carbon dioxide in a first reactor, particularly a pressure regulated first reactor, to obtain a carbon dioxide releasing carrier material,
   (b) transferring the carbon dioxide releasing carrier material to a second reactor, particularly a pressure regulated second reactor,
   (c) dehydrogenating the carbon dioxide releasing carrier material in a second reactor to thereby obtain compressed products comprising pressurized carbon dioxide and the carrier material, wherein the carrier material is hydrogen,
   (d) separating the compressed products comprising pressurized carbon dioxide and the carrier material obtained in step (c) to obtain pressurized carbon dioxide, and
   (e) optionally supplying the first reactor with the carrier material, such as the separated carrier material, obtained in step (d) for the reaction, such as reducing, particularly hydrogenating, electrochemically reducing and/or hydrothermal reducing, in step (a).
3. The method according to any one of the preceding aspects, wherein the releasing (c) is carried out under pressure, particularly at a pressure as defined in aspect 18, and the pressure is maintained by the release of the carbon dioxide and/or carrier material from the carbon dioxide releasing carrier material.
4. The method according to any one of the preceding aspects, wherein the compression is achieved by increasing the pressure of carbon dioxide releasing carrier material in step (b), particularly by compression means such as by a pump, and/or wherein the compression is achieved by adjusting the pressure of carbon dioxide releasing carrier material in step (b), particularly by compression means such as by a pump, to the pressure in the second reactor, and/or wherein the compression is achieved by the releasing of the carbon dioxide and/or carrier material from the carbon dioxide releasing carrier material in step (c).
5. The method according to any one of the preceding aspects, wherein the releasing in step (c) and/or the transfer in step (b) is/are the only means for the compression of the pressurized carbon dioxide.
6. The method according to any one of the preceding aspects, wherein step (a) further includes
   (A) the continuous supply of carbon dioxide and/or the continuous generation of carbon dioxide in the first reactor, and/or
   (B) the continuous supply of carrier material, such as H₂O or H₂, and/or the continuous generation of carrier material, such as H₂O or H₂, in the first reactor, and/or
   wherein step (c) further includes the continuous supply of carbon dioxide releasing carrier material to the second reactor.
7. The method according to any one of the preceding aspects, wherein step (a) further includes the purification of the carbon dioxide releasing carrier material, particularly before transferring the carbon dioxide releasing carrier material into the second reactor.
8. The method according to any one of the preceding aspects, wherein step (e) includes the supply with further carrier material, and/or step (b) includes the supply with further carbon dioxide releasing carrier material.
9. The method according to any one of the preceding aspects, wherein the method further comprises the step (f1) collecting the pressurized carbon dioxide, preferably in pressure tanks, (f2) using the pressurized carbon dioxide for chemical reactions, (f3) feed the pressurized carbon dioxide into carbon dioxide pipelines or carbon dioxide supply networks e.g. for transportation or storage of carbon dioxide into underground or sub-sea level CO₂ deposits (i.e. CCS), (f4) collecting the separated and compressed carrier material, such as H₂, preferably in pressure tanks, (f5) using the separated and compressed carrier material, such as H₂, for chemical reactions, and/or (f6) feed the separated and compressed carrier material, such as H₂, into hydrogen pipelines or hydrogen supply networks.
10. The method according to any one of the preceding aspects, wherein the carbon dioxide releasing carrier material is a fluid such as a liquid or gas, preferably the carbon dioxide releasing carrier material is a liquid.
11. The method according to any one of the preceding aspects, wherein the step of transferring the carbon dioxide releasing carrier material (b) includes:
   (b1) increasing the pressure of the carbon dioxide releasing carrier material so that the carbon dioxide releasing carrier material is supplied to the second reactor at a pressure of 10 to 4000 bar, such as 50 to 2000 bar, 100 to 1000 bar, 400 to 1000 bar or 600 to 900 bar,
   (b2) increasing the pressure of the carbon dioxide releasing carrier material to a pressure used in the second reactor for the releasing in step (c), and/or
   (b3) receiving the carbon dioxide releasing carrier material from the first reactor having a first pressure, preferably 1 to 100 bar, such as 1 to 50 bar, 5 to 50, 10 to 40 bar or 20 to 40 bar, and increasing the pressure of the carbon dioxide releasing carrier material to a second pressure, preferably 10 to 4000 bar, such as 50 to 2000 bar, 100 to 1000 bar, 74 to 1000 bar, 400 to 1000 bar or 600 to 900 bar, wherein the second pressure is higher than the first pressure,
      wherein preferably a pump is used in options (b1), (b2) and/or (b3) to increases the pressure.
12. The method according to any one of the preceding aspects, wherein the release in step (c) is a dehydrogenation of a carbon dioxide releasing carrier material, such as formic acid, to thereby obtain compressed products comprising pressurized carbon dioxide and a carrier material that is H₂.
13. The method according to any one of the preceding aspects, wherein the electrochemically reduction in step (a) is carried out using carbon dioxide and, H₂O and/or H₂ as a carrier material, to obtain formic acid, methanol, or alcohols as the carbon dioxide releasing carrier material, preferably the electrochemically reducing in step (a) is carried out using carbon dioxide and H₂O to obtain formic acid as the carbon dioxide releasing carrier material.
14. The method according to any one of the preceding aspects, wherein the reaction in step (a) is carried out using a catalyst, preferably a ruthenium catalyst, palladium catalyst, molybdenum catalyst and/or iridium catalyst, wherein preferably the ligands of the catalyst are amine-based ligands such as pyridine-based ligands, imidazole-based ligands, 2-imidazoline-based ligands or pyrimidine-based ligands, and/or phosphorus-based ligands such as phosphine ligands, or combinations thereof.
15. The method according to any one of the preceding aspects, wherein the releasing in step (c) is carried out using a catalyst, preferably a ruthenium catalyst, palladium catalyst, molybdenum catalyst and/or iridium catalyst, wherein preferably the ligands of the catalyst are amine-based ligands such as pyridine-based ligands, imidazole-based ligands, 2-imidazoline-based ligands or pyrimidine-based ligands, and/or phosphorus-based ligands such as phosphine ligands, or combinations thereof.
16. The method according to any one of the preceding aspects, wherein the releasing in step (c) and/or reaction, such as reducing, particularly hydrogenating, electrochemically reducing and/or hydrothermal reducing, in step (a) is/are carried out using heterogenous and/or homogenous catalyst, preferably a fixed-bed catalyst.
17. The method according to any one of the preceding aspects, wherein the reaction, such as reducing, particularly hydrogenating, electrochemically reducing and/or hydrothermal reducing, in step (a) in the first reactor is carried out
   (i) at a pressure of 1 to 1000 bar, such as 1 to 100 bar, 1 to 50 bar, 5 to 50, 10 to 40 bar or 20 to 40 bar, and/or
   (ii) at a temperature of 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C.
18. The method according to any one of the preceding aspects, wherein the releasing in step (c) in the second reactor is carried out
   (i) at a pressure of 10 to 4000 bar, such as 50 to 2000 bar, 100 to 1000 bar, 400 to 1000 bar or 600 to 900 bar, preferably the pressure in the second reactor is higher than the pressure in the first reactor and/or
   (ii) at a temperature of 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C.
19. The method according to any one of the preceding aspects, wherein the pressure of the second rector is controlled by pressure control means and/or a pressure control outlet for the compressed products and/or the pressurized carbon dioxide, such as a pressure valve outlet.
20. The method according to any one of the preceding aspects, wherein the pressure of the pressurized carbon dioxide is 10 to 4000 bar, such as 50 to 2000 bar, 100 to 1000 bar, 400 to 1000 bar or 600 to 900 bar.
21. The method according to any one of the preceding aspects, wherein the carrier material for the reaction, such as reducing, particularly hydrogenating, electrochemically reducing and/or hydrothermal reducing, in step (a) is dissolved in a solvent and/or wherein the carbon dioxide releasing carrier material for the releasing in step (c) is dissolved in a solvent, wherein the solvent is preferably water, ionic liquids, 1,4-dioxane, dimethyl sulfoxide (DMSO), alcohols such as methanol and/or N,N-dimethylformamide (DMF).
22. The method according to aspect 21, wherein the solvent further comprises a base, such as NEt₃, 1,8-diazabicyclo[5.4.0]undec-7-ene (DBU), KOH, KHCO₃, HCO₂Na, NHex₃ and/or triacetonamines, preferably 2,2,6,6-Tetramethyl-4-piperidone (TAA), n-butyl triacetonediamine (n-Bu TAD), 4-amino-2,2,6,6-tetramethylpiperidine (TAD), N,N-dimethyl-N'-(2,2,6,6-tetramethylpiperidin-4-yl)propane-1,3-diamine (TAT), 4-dimethylamino-2,2,6,6-tetramethylpiperidine (DMA-TEMP), N,N'-bis(2,2,6,6-tetramethylpiperidin-4-yl)hexane-1,6-diamine (HMBTAD).
23. The method according to any one of the preceding aspects, wherein step (b) and/or (c) further includes that solvent, such as 1 to 100 wt.%, 10 to 99 wt.%, 50 to 95 wt.%, 10 to 50 wt.%, 10 to 20 wt.%, or 30 to 50 wt.% or 80 to 90 wt.% of the solvent, is separated and returned to the first reactor, particularly solvent is separated and returned to the first reactor before entering the second reactor, preferably before entering the pump and/or the solvent is separated and returned from the second reactor to the first reactor, and/or wherein the transfer in step (b) includes the solvent in which the carbon dioxide releasing carrier material is dissolved.
24. The method according to any one of the preceding aspects, wherein the molar ratio of carrier material, such as H₂, to the carbon dioxide, is 1/10 to 10/1, such as 1/1 to 10/1, 1/1 to 1/10,2/1 to 1/2, 1/1 to 3/1, or 1/1 to 1/3.
25. The method according to any one of the preceding aspects, wherein the carbon dioxide releasing carrier material is formic acid, formic acid salts (formates), formaldehyde, amine stabilized formic acid, amine stabilized formaldehyde, methanol, alcohols, carboxylic acids, aldehydes, and/or alkyl formates, preferably formic acid.
26. The method according to any one of the preceding aspects, wherein the method further comprises (h) purifying the pressurized hydrogen and/or pressurized carbon dioxide that are separated in step (d) using a membrane, an alkaline gas scrubber, electrochemical separation, condenser and/or amine gas treating.
27. The method according to any one of the preceding aspects, wherein the separation in step (d) includes the condensation of the carbon dioxide, and/or separating using a membrane, an alkaline gas scrubber, electrochemical separation, and/or amine gas treating, preferably the separation in step (d) includes the condensation of the carbon dioxide.
28. The method according to any one of the preceding aspects, wherein the supply in step (e) further includes the adjustment of the pressure of the carrier material to the pressure in the first reactor such as a pressure as defined in aspect 17, preferably using a device for pressure control, particularly a pressure control outlet such as pressure control valve, flash evaporation chamber, a device for energy recovery such as an expander/turbine and/or a heat exchanger, and/or wherein the pressure of the separated pressurized carbon dioxide is adjusted to a pressure as defined in aspect 18, preferably using a device for pressure control, particularly a pressure control outlet such as pressure control valve, flash evaporation chamber, a device for energy recovery such as an expander/turbine and/or a heat exchanger.
29. The method according to aspect 28, wherein the carrier material is heated or cooled after the adjustment of the pressure, preferably to a temperature as defined in aspect 17, more preferably to a temperature used for the reaction, such as reducing, particularly hydrogenating, electrochemically reducing and/or hydrothermal reducing, in step (a) in the first reactor, and/or wherein the pressurized hydrogen is heated or cooled after the adjustment of the pressure, preferably to 10 to 50 °C, such as 10 to 40 °C, 15 to 30 °C.
30. A reactor system for the continuous production of pressurized carbon dioxide, preferably using the method as defined in any one of the preceding method aspects, preferably according to the use as defined in aspects 46 to 49, comprising:
   (a) a first reactor, particularly a pressure regulated first reactor, for the reaction, such as reduction, particularly hydrogenation, electrochemically reduction and/or hydrothermal reduction, of carbon dioxide to obtain a carbon dioxide releasing carrier material,
   (b) a first connecting element for the transfer of the carbon dioxide releasing carrier material from the first reactor to a second reactor,
   (c) a second reactor, particularly a pressure regulated second reactor, for the releasing of the carbon dioxide from the carbon dioxide releasing carrier material to thereby obtain compressed products comprising pressurized carbon dioxide and the carrier material,
   (d) a separator in connection with the second reactor (c) for the separation of the compressed products comprising the pressurized carbon dioxide and the carrier material,
   (e) optionally a second connecting element connected to the separator for the transfer of the carrier material from the separator (d) to the first reactor (a).
31. The reactor system according to aspect 30, wherein the first reactor (a) and the second reactor (c) are directly and/or physically connected by the first connecting element (c), such as a conduit, and/or second connecting element (e), such as a conduit.
32. The reactor system according to any one of aspects 30 or 31, wherein the reactor system does not comprise a pressure pump for the compression of the pressurized carbon dioxide and/or carbon dioxide.
33. The reactor system according to any one of aspects 30 to 32, wherein the first connecting element (b) comprises a pressure pump, such as a radial, axial, piston, and/or membrane pump, to adjust/increase the pressure to the pressure in the second reactor (c), particularly adjust/increase the pressure to 10 to 4000 bar, such as 50 to 2000 bar, 100 to 1000 bar, 400 to 1000 bar or 600 to 900 bar.
34. The reactor system according to any one of aspects 30 to 33, wherein the second reactor (c) for the releasing carbon dioxide from the carbon dioxide releasing carrier material and/or a pressure pump arranged within the connecting element (b) for the transfer of the carbon dioxide releasing carrier material to a second reactor is the only means for the compression of the pressurized carbon dioxide.
35. The reactor system according to any one of aspects 30 to 34, wherein the reactor system further comprises a third connecting element for the transfer of a solvent from the second reactor (a) to the first reactor (c).
36. The reactor system according to any one of aspects 30 to 35, wherein the reactor system further comprises a device for pressure control, particularly a pressure control outlet such as pressure control valve, and/or a device for energy recovery such as an expander/turbine and/or a heat exchanger, a pressure control valve and/or flash evaporation chamber, preferably located between the separator (d) and the first reactor (a) and arranged within the second connecting element (e), arranged between the separator (d) and the second reactor (c), and/or connected to the separator (d) for the separated and pressurized carbon dioxide and/or the separated and pressurized carrier material, such as hydrogen.
37. The reactor system according to any one of aspects 30 to 36, wherein the reactor system further comprises a carbon dioxide purification chamber for purifying the pressurized carbon dioxide using a membrane, electrochemical separation, condenser, an alkaline gas scrubber and/or amine gas treating, wherein the carbon dioxide purification chamber is connected to the separator (d) and receives the pressurized carbon dioxide after the separation.
38. The reactor system according to any one of aspects 30 to 37, wherein the reactor system further comprises a hydrogen purification chamber for purifying the pressurized hydrogen (i.e the separated and pressurized hydrogen) using a membrane, electrochemical separation, condenser, an alkaline gas scrubber and/or amine gas treating, wherein the hydrogen purification chamber is connected to the separator (d) and receives the pressurized hydrogen after the separation.
39. The reactor system according to any one of aspects 30 to 38, wherein the separator (d) is a condenser, particularly a CO₂ condenser, a separator comprising a membrane for the separation, an alkaline gas scrubber and/or amine gas treating separator, and/or an electrochemical separator.
40. The reactor system according to any one of aspects 30 to 39, wherein the volume of the first reactor (a) is 100 mL to 2.000.000 L, such as 1 L to 200.000 L, 100 L to 100.000 L or 1.000 L to 50.000 L, and/or the volume of the second reactor (c) is 100 mL to 2.000.000 L, such as 1 L to 200.000 L, 100 L to 100.000 L or 1.000 L to 50.000 L.
41. The reactor system according to any one of aspects 30 to 40, wherein the first reactor (a) and/or the second reactor (b) have a cylindrical shape.
42. The reactor system according to any one of aspects 30 to 41, wherein the second reactor (c) comprises pressure control means and/or a pressure control outlet for the compressed products and/or the pressurized carbon dioxide, such as a pressure valve outlet, and the separator (d) is connected via the pressure valve outlet with the second reactor (c).
43. The reactor system according to any one of aspects 30 to 42, wherein the reactor system comprises pressure control means or a pressure control outlet for the pressurized carbon dioxide, preferably connected to the separator (d) or a carbon dioxide purification chamber as defined in aspect 37.
44. The reactor system according to any one of aspects 30 to 43, wherein the reactor system further comprises a pressure tank, carbon dioxide loading station, fueling station, pipeline, and/or supply network for receiving the pressurized carbon dioxide connected to the separator (d), and/or a carbon dioxide purification chamber as defined in aspect 37, and/or for receiving the pressurized hydrogen connected to the separator (d), and/or a carbon dioxide purification chamber as defined in aspect 37.
45. The reactor system according to any one of aspects 30 to 44, wherein the reactor system further comprises at least one buffer tank for the carrier material located between the separator (d) and the first reactor (a) and arranged within the second connecting element (e) and/or wherein the reactor system further comprises at least one buffer tank for the carbon dioxide releasing carrier material located between the first reactor (a) and the second reactor (c) and arranged within the first connecting element (b).
46. Use of a carrier material for the continuous production of pressurized carbon dioxide, preferably the continuous production of pressurized carbon dioxide as defined according to any one of the preceding method aspects and/or preformed in a reactor system according to any one of the preceding reactor system aspects, wherein the continuous production of pressurized carbon dioxide includes:
   (a) the release of the carbon dioxide from a carbon dioxide releasing carrier material to thereby obtain compressed products comprising pressurized carbon dioxide and the carrier material, and
   (b) the reaction, such as reduction, particularly hydrogenation, electrochemically reduction and/or hydrothermal reduction, of the carrier material obtained in the release step (a) to obtain the carbon dioxide releasing carrier material used for the release step (a).
47. Use according to aspect 46, wherein the compression is achieved by the release of carbon dioxide and/or carrier material, such as hydrogen, from the carbon dioxide releasing carrier material in step (a), wherein the release (a) is carried out under pressure, particularly at a pressure as defined in aspect 18, and/or the pressure is maintained by the release of carbon dioxide and/or carrier material, such as hydrogen, from the carbon dioxide releasing carrier material, particularly the pressure is maintained by releasing carbon dioxide from and/or carrier material, such as hydrogen, the carbon dioxide releasing carrier material during the release step.
48. Use according to any one of aspect 46 or 47, wherein the compression is achieved and/or maintained by releasing carbon dioxide gas or supercritical carbon dioxide from a carbon dioxide releasing carrier material, such as a liquid carbon dioxide releasing carrier material and/or a substantially non-compressible carbon dioxide releasing carrier material, in a pressure regulated second reactor during the release in step (a), and/or wherein the compression is achieved and/or maintained by releasing hydrogen gas or supercritical hydrogen from a carbon dioxide releasing carrier material, such as a liquid carbon dioxide releasing carrier material and/or a substantially non-compressible carbon dioxide releasing carrier material, in a pressure regulated second reactor during the release in step (a).
49. Use according to any one of aspect 46 to 48, wherein the carbon dioxide releasing carrier, preferably liquid carbon dioxide releasing carrier, is continuously transferred to the release step (a).

## Claims

1. A method for the continuous production of pressurized carbon dioxide comprising:
(a) reacting, such as reducing, particularly hydrogenating, electrochemically reducing and/or hydrothermal reducing, the carbon dioxide in a first reactor, to obtain a carbon dioxide releasing carrier material,
(b) transferring the carbon dioxide releasing carrier material to a second reactor,
(c) releasing carbon dioxide from the carbon dioxide releasing carrier material in a second reactor to thereby obtain compressed products comprising pressurized carbon dioxide and the carrier material,
(d) separating the compressed products comprising pressurized carbon dioxide and the carrier material obtained in step (c) to obtain pressurized carbon dioxide, and
(e) optionally supplying the first reactor with the carrier material, such as the separated carrier material, obtained in step (d) for the reaction in step (a).

2. The method according to claim 1, wherein the compression is achieved by increasing the pressure of carbon dioxide releasing carrier material in step (b), particularly by compression means such as by a pump, and/or wherein the compression is achieved by adjusting the pressure of carbon dioxide releasing carrier material in step (b), particularly by compression means such as by a pump, to the pressure in the second reactor, and/or wherein the compression is achieved by the releasing of the carbon dioxide and/or carrier material from the carbon dioxide releasing carrier material in step (c).

3. The method according to any one of the claims 1 or 2, wherein the step of transferring the carbon dioxide releasing carrier material (b) includes:
(b1) increasing the pressure of the carbon dioxide releasing carrier material so that the carbon dioxide releasing carrier material is supplied to the second reactor at a pressure of 10 to 4000 bar, such as 50 to 2000 bar, 100 to 1000 bar, 400 to 1000 bar or 600 to 900 bar,
(b2) increasing the pressure of the carbon dioxide releasing carrier material to a pressure used in the second reactor for the releasing in step (c), and/or
(b3) receiving the carbon dioxide releasing carrier material from the first reactor having a first pressure, preferably 1 to 100 bar, such as 1 to 50 bar, 5 to 50, 10 to 40 bar or 20 to 40 bar, and increasing the pressure of the carbon dioxide releasing carrier material to a second pressure, preferably 10 to 4000 bar, such as 50 to 2000 bar, 100 to 1000 bar, 400 to 1000 bar or 600 to 900 bar, wherein the second pressure is higher than the first pressure,
wherein preferably a pump is used in options (b1), (b2) and/or (b3) to increases the pressure.

4. The method according to any one of the preceding claims, wherein the electrochemically reduction in step (a) is carried out using carbon dioxide and H₂O and/or H₂ as a carrier material to obtain formic acid, methanol, or alcohols as the carbon dioxide releasing carrier material, preferably the electrochemically reducing in step (a) is carried out using carbon dioxide and H₂O to obtain formic acid as the carbon dioxide releasing carrier material.

5. The method according to any one of the preceding claims,
(A) wherein the reaction, such as reducing, particularly hydrogenating, electrochemically reducing and/or hydrothermal reducing, in step (a) in the first reactor is carried out
(i) at a pressure of 1 to 1000 bar, such as 1 to 100 bar, 1 to 50 bar, 5 to 50, 10 to 40 bar or 20 to 40 bar, and/or
(ii) at a temperature of 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C, and/or
(B) wherein the release in step (c) in the second reactor is carried out
(i) at a pressure of 10 to 4000 bar, such as 50 to 2000 bar, 100 to 1000 bar, 400 to 1000 bar or 600 to 900 bar, preferably the pressure in the second reactor is higher than the pressure in the first reactor and/or
(ii) at a temperature of 20 to 500 °C, such as 30 to 400 °C, 50 to 300 °C, 50 to 200 °C, 80 to 150 °C, 90 to 150 °C, 20 to 150 °C, 20 to 80 °C, 20 to 50°C or 100 to 130 °C.

6. The method according to any one of the preceding claims, wherein the pressure of the second rector is controlled by pressure control means and/or a pressure control outlet for the compressed products and/or the pressurized carbon dioxide, such as a pressure valve outlet.

7. The method according to any one of the preceding claims,
(i) wherein the carrier material for reaction, such as reducing, particularly hydrogenating, electrochemically reducing and/or hydrothermal reducing, in step (a) is dissolved in a solvent and/or wherein the carbon dioxide releasing carrier material for the releasing in step (c) is dissolved in a solvent, wherein the solvent is preferably water, ionic liquids, 1,4-dioxane, dimethyl sulfoxide (DMSO), alcohols such as methanol and/or N,N-dimethylformamide (DMF), and/or
(ii) wherein step (b) and/or (c) further includes that solvent, such as 1 to 100 wt.%, 10 to 99 wt.%, 50 to 95 wt.%, 10 to 50 wt.%, 10 to 20 wt.%, or 30 to 50 wt.% or 80 to 90 wt.% of the solvent, is separated and returned to the first reactor, particularly solvent is separated and returned to the first reactor before entering the second reactor, preferably before entering the pump, and/or the solvent is separated and returned from the second reactor to the first reactor, and/or wherein the transfer in step (b) includes the solvent in which the carbon dioxide releasing carrier material is dissolved.

8. The method according to any one of the preceding claims, wherein the carbon dioxide releasing carrier material is formic acid, formic acid salts (formates), formaldehyde, amine stabilized formic acid, amine stabilized formaldehyde, methanol, alcohols, carboxylic acids, aldehydes, and/or alkyl formates, preferably formic acid.

9. The method according to any one of the preceding claims, wherein the supply in step (e) further includes the adjustment of the pressure of the carrier material to the pressure in the first reactor such as a pressure as defined in claim 5 (A), preferably using a device for pressure control, particularly a pressure control outlet such as pressure control valve, flash evaporation chamber, a device for energy recovery such as an expander/turbine and/or a heat exchanger, and/or wherein the pressure of the separated pressurized carbon dioxide is adjusted to a pressure as defined in claim 5 (B), preferably using a device for pressure control, particularly a pressure control outlet such as pressure control valve, flash evaporation chamber, a device for energy recovery such as an expander/turbine and/or a heat exchanger.

10. A reactor system for the continuous production of pressurized carbon dioxide comprising:
(a) a first reactor for the reaction, such as reduction, particularly hydrogenation, electrochemically reduction and/or hydrothermal reduction, of carbon dioxide to obtain a carbon dioxide releasing carrier material,
(b) a first connecting element for the transfer of the carbon dioxide releasing carrier material from the first reactor to a second reactor,
(c) a second reactor for the releasing of the carbon dioxide from the carbon dioxide releasing carrier material to thereby obtain compressed products comprising pressurized carbon dioxide and the carrier material,
(d) a separator in connection with the second reactor (c) for the separation of the compressed products comprising the pressurized carbon dioxide and the carrier material,
(e) optionally a second connecting element connected to the separator for the transfer of the carrier material from the separator (d) to the first reactor (a).

11. The reactor system according to claim 10, wherein the first reactor (a) and the second reactor (c) are directly and/or physically connected by the first connecting element (c), such as a conduit, and/or second connecting element (e), such as a conduit.

12. The reactor system according to any one of claim 10 or 11
(A) wherein the first connecting element (b) comprises a pressure pump, such as a radial, axial, piston, and/or membrane pump, to adjust/increase the pressure to the pressure in the second reactor (c), particularly adjust/increase the pressure to 10 to 4000 bar, such as 50 to 2000 bar, 100 to 1000 bar, 400 to 1000 bar or 600 to 900 bar,
(B) wherein the second reactor (c) for releasing carbon dioxide from the carbon dioxide releasing carrier material and/or a pressure pump arranged within the connecting element (b) for the transfer of the carbon dioxide releasing carrier material to a second reactor is the only means for the compression of the pressurized carbon dioxide, and/or
(C) wherein the second reactor (c) comprises pressure control means and/or a pressure control outlet for the compressed products and/or the pressurized carbon dioxide, such as a pressure valve outlet, and the separator (d) is connected via the pressure valve outlet with the second reactor (c).

13. The reactor system according to any one of claims 10 to 12, wherein the reactor system further comprises
(I) a third connecting element for the transfer of a solvent from the second reactor (a) to the first reactor (c),
(II) a device for pressure control, particularly a pressure control outlet such as pressure control valve, and/or a device for energy recovery such as an expander/turbine and/or a heat exchanger, a pressure control valve and/or flash evaporation chamber, preferably located between the separator (d) and the first reactor (a) and arranged within the second connecting element (e), arranged between the separator (d) and the second reactor (c), and/or connected to the separator (d) for the separated and pressurized carbon dioxide and/or the separated and pressurized carrier material, such as hydrogen, and/or
(III) pressure control means or a pressure control outlet for the pressurized carbon dioxide, preferably connected to the separator (d) or a carbon dioxide purification chamber for purifying the pressurized carbon dioxide using a membrane, wherein the carbon dioxide purification chamber is connected to the separator (d) and receives the pressurized carbon dioxide after the separation.

14. Use of a carrier material for the continuous production of pressurized carbon dioxide, wherein the continuous production of pressurized carbon dioxide includes:
(a) the release of the carbon dioxide from a carbon dioxide releasing carrier material to thereby obtain compressed products comprising pressurized carbon dioxide and the carrier material, and
(b) the reaction, such as reduction, particularly hydrogenation, electrochemically reduction and/or hydrothermal reduction, of the carrier material obtained in the release step (a) to obtain the carbon dioxide releasing carrier material used for the release step (a).

15. Use according to claim 14, wherein the compression
(A) is achieved by the release of carbon dioxide and/or carrier material, such as hydrogen, from the carbon dioxide releasing carrier material in step (a), wherein the release (a) is carried out under pressure, and/or the pressure is maintained by the release of carbon dioxide and/or carrier material, such as hydrogen, from the carbon dioxide releasing carrier material, particularly the pressure is maintained by releasing carbon dioxide from and/or carrier material, such as hydrogen, the carbon dioxide releasing carrier material during the release step, and/or
(B) is achieved and/or maintained by releasing carbon dioxide gas or supercritical carbon dioxide from a carbon dioxide releasing carrier material, such as a liquid carbon dioxide releasing carrier material and/or a substantially non-compressible carbon dioxide releasing carrier material, in a pressure regulated second reactor during the release in step (a).
